# EUROPÄISCHE PATENTANMELDUNG

(11) **EP 3 037 513 A1**
(43) Veröffentlichungstag der Anmeldung: **29.06.2016**
(21) Anmeldenummer: 15167518.8
(22) Anmeldetag: 13.05.2015
(51) Int. Cl.: C12M 1/00

(54) **VERFAHREN ZUR KONTINUIERLICHEN ELUTION EINES PRODUKTES VON CHROMATOGRAPHIESÄULEN**

(71) Anmelder: Bayer Technology Services GmbH, 51368 Leverkusen (DE)
(72) Erfinder: Schwan, Peter, 51373 Leverkusen (DE); Baumarth, Kerstin, 42329 Wuppertal (DE); Lobedann, Martin, 51069 Köln (DE)
(74) Vertreter: BIP Patents

(57) **Zusammenfassung**

Die Erfindung stellt ein Verfahren zur kontinuierlichen Elution eines biopharmazeutischen, biologischen, makromolekularen Produktes von mehr als einer Chromatographiesäule zur Verfügung, umfassend die Schritte:
(a) Bereitstellen eines Produktstromes über einen Inlet,
(b) gleichzeitiges Beladen von n Front-Chromatographiesäulen mit dem Produktstrom in einer Beladungszone mit einer Beladungszeit t_{B}, wobei ein Ausgangsstrom der n Front-Chromatographiesäulen auf mindestens n-1 Recovery-Chromatographiesäulen in einer Recoveryzone gleichzeitig verteilt wird,
wobei n zwischen 1 und 5 liegt, und
wobei die n Front-Chromatographiesäulen zu einem gegebenen Zeitpunkt eine unterschiedlich lange Beladungszeit t_{B} zwischen 0 und L₁, zwischen L₁ und L₂, und bis zu zwischen Ln-1 und Ln aufweisen,
wobei jede Front-Chromatographiesäule eine gesamte Beladungszeit von Lₙ aufweist, dadurch gekennzeichnet, dass periodisch nach einer konstanten Switch Zeit ts von Lₙ - Lₙ₋₁, die Front-Chromatographiesäulen n aus der Beladungszone heraustritt und eine Recovery-Chromatographiesäule aus der Recoveryzone in die Beladungszone eintritt,
(c) Waschen der mit Produkt beladenen Säule aus Schritt (b) mit mindestens einem Waschpuffer,
(d) Elution des Produktes von der gewaschenen Säule aus Schritt (c) mit einer Elutionszeit t_{E}, die ≥ 80% der Switch-Zeit ts ist, und

dass sich über 80% der Gesamtlaufzeit t_{G} kontinuierlich mindestens eine Chromatographiesäule im Elutionsschritt (d) befindet.

## Beschreibung

Die Erfindung betrifft ein Verfahren zur kontinuierlichen Elution eines Produktes von Chromatographiesäulen.

Gewöhnlich werden Proteine in der biotechnologischen Herstellung im Batch aufgereinigt (englisch *batch*: "Stapel"). Das bedeutet, dass die einzelnen Produktionszyklen chargenweise, diskontinuierlich gehandhabt werden, wobei nach dem Abschluss eines Produktionszyklus das Produkt als Ganzes entnommen wird. Für eine erneute Produktion muss dann separat ein neuer Produktzyklus bzw. Batch gestartet werden.

In den letzten Jahren hat sich nun mehr und mehr gezeigt, dass eine kontinuierliche Verfahrensweise auch in der biotechnologischen Herstellung gefahren werden kann, wobei das Verfahren im Gegensatz zum Batch-Verfahren ohne Unterbrechungen laufen kann.

Die stark regulierte pharmazeutische Produktion erfordert einen großen zeitlichen, technischen und personellen Aufwand für die Bereitstellung gereinigter und sterilisierter Bioreaktoren und Gewährleistung eines keimfreien Produkts. Um Kreuzkontaminationen bei einem Produktwechsel in einer Multi-Purpose-Anlage oder zwischen zwei Produktchargen sicher zu vermeiden, wird außer der Reinigung eine sehr aufwändige Reinigungsvalidierung benötigt, welche bei einer Prozessadaption ggf. wiederholt werden muss.
Dies gilt sowohl für das Upstream-Processing (USP), d.h. die Herstellung biologischer Produkte in Fermentern als auch für das Downstream-Processing (DSP), d. h. die Aufreinigung der Fermentationsprodukte.
Gerade bei der Fermentation ist eine keimfreie Umgebung für eine erfolgreiche Kultivierung essentiell. Zur Sterilisation von Batch- oder Fedbatch-Fermentern kommt in der Regel die SIP-Technik zum Einsatz (SIP = sterilization-in-place).
Der durch die Bereitstellungsprozeduren bedingte Nutzungsausfall der Reaktoren kann insbesondere bei kurzen Nutzungsperioden und häufigem Produktwechsel in der Größenordnung der Reaktorverfügbarkeit liegen. Betroffen sind im USP der biotechnologischen Produktion z. B. die Prozessschritte der Medienherstellung und Fermentation und im DSP das Solubilisieren, Einfrieren, Auftauen, pH-Adjustieren, Produkttrennung wie z. B. durch Chromatographie, Fällen, oder Kristallisieren, das Umpuffern und die Virusinaktivierung.

Die regulatorischen Anforderungen sind dabei im Downstream-Bereich eine keimarme Prozessführung. Daher ist eine sterile Fahrweise im Falle des Batch-Betriebes nicht gefordert. In einem kontinuierlichen Verfahren jedoch wird die Reinigung des Proteins über einen längeren Zeitraum möglichst ohne Reinigungsschritte betrieben. Dies geschieht bevorzugt ohne Sterilisationsschritte während der Reinigung. Dabei ist aber das Verkeimungsrisiko um ein Vielfaches höher als bei einem reinen Batchbetrieb.

WO2012/078677 beschreibt ein Verfahren und eine Anlage zur kontinuierlichen Aufbereitung von biopharmazeutischen Produkten durch Chromatographie und deren Integration in einer Produktionsanlage insbesondere in einer Einweg-Anlage. Obwohl WO2012/078677 Ansätze zur kontinuierlichen Produktion von biopharmazeutischen und biologischen Produkten liefert, reicht die offenbarte Lösung in der Praxis nicht aus. WO2012/078677 offenbart auch nicht die kontinuierliche Elution von Produkt.

US 2014/0255994 A1 offenbart ein integriertes kontinuierliches Verfahren zur Herstellung von therapeutischen Proteinen. US 2014/0255994 A1 offenbart allerdings nicht das Merkmal, dass in so einem Verfahren kontinuierlich eluiert werden kann.

EP 2 182 990 A1 offenbart ein Verfahren zur Sterilisation von Chromatographiesäulen durch Verwendung von heißem Wasserdampf.

Zunächst seien einige Begriffe näher definiert.

Ein kontinuierliches Verfahren, bzw. kontinuierliche Elution meint im Rahmen der Erfindung jeden Prozess zur Durchführung von mindestens zwei Prozessschritten in Serie, in dem der Ausgangsstrom eines vorgeschalteten Schrittes in einen nachgeschalteten Schritt befördert wird. Der nachgeschaltete Schritt beginnt die Bearbeitung des Produktstroms, bevor der vorgeschaltete Schritt abgeschlossen ist. Üblicherweise wird in einem kontinuierlichen Verfahren immer ein Teil des Produktstroms in der Produktionsanlage befördert und wird als "kontinuierlicher Produktstrom" bezeichnet. Entsprechend bedeutet eine kontinuierliche Beförderung oder Transfer eines Produktstroms von einer vorgeschalteten Unit in eine nachgeschaltete Unit, dass die nachgeschaltete Unit bereits arbeitet, bevor die vorgeschaltete Unit außer Betrieb genommen wird, d.h. dass zwei nacheinander geschaltete Units den Produktstrom gleichzeitig prozessieren, der sie durchfließt.

Der Begriff "Front-Chromatographiesäule" bedeutet im Rahmen der Erfindung eine Chromatographiesäule, die in einer seriellen Verschaltung während der Beladung in der ersten Position steht, und direkt mit Produktstrom beladen wird.

Der Begriff "Recovery-Chromatographiesäule" bedeutet im Rahmen der Erfindung eine Chromatographiesäule, die in einer seriellen Verschaltung während der Beladung in der zweiten Position hinter den Front-Chromatographiesäulen steht und mit dem Produktstrom aus den Front-Chromatographiesäulen beladen wird.

Die "Beladungszeit t_{B}" bedeutet im Rahmen der Erfindung die Zeit, in der sich eine Chromatographiesäule als "Front-Chromatographiesäule in der Beladungszone befindet.

Die "Switch Zeit ts" bedeutet im Rahmen der Erfindung, dass periodisch nach einer konstanten Switch Zeit ts die Front-Chromatographiesäulen n aus der Beladungszone heraustritt und eine Recovery-Chromatographiesäule aus der Recoveryzone in die Beladungszone eintritt.

Die "Elutionszeit t_{E}" bedeutet im Rahmen der Erfindung die Zeit, in der das Produkt durch einen Elutionspuffer von einer Chromatographiesäule in den Produktausgang eluiert wird.

Die "Gesamtlaufzeit t_{G}" bedeutet im Rahmen der Erfindung die Zeit, für welche das erfindungsgemäße Verfahren insgesamt ohne Pause betrieben wird.

Der Begriff "keimreduziert" meint im Rahmen der Erfindung einen Zustand reduzierter Keimzahl, also einer Zahl an Mikroorganismen pro Flächen- oder Volumeneinheit von nahezu Null, der durch eine geeignete Keimreduktionsmethode erreichbar ist, wobei diese Keimreduktionsmethode ausgewählt sein kann aus Gamma-Bestrahlung, Beta-Bestrahlung, Autoklavieren, Ethylenoxid (ETO)-Behandlung und "steam-in-place" (SIP)-Behandlung.

Der Begriff "Einwegartikel" bedeutet im Rahmen der Erfindung, dass die betreffenden produkt-berührten Teile, insbesondere Apparaturen, Behältern, Filter und Verbindungselemente, zum einmaligen Gebrauch mit anschließendem Wegwerfen geeignet sind (Englisch "disposable"), wobei diese Behälter sowohl aus Kunststoff als auch aus Metall sein können. Im Rahmen der Erfindung umfasst der Begriff auch Mehrwegartikel, etwa aus Stahl, die im erfindungsgemäßen Verfahren nur einmal verwendet werden und dann im Verfahren nicht mehr zum Einsatz kommen. Diese Mehrwegartikel, z.B. aus Stahl, werden dann im Rahmen der Erfindung auch "als Einwegartikel benutzte" Gegenstände bezeichnet. Solche eingesetzten Einwegartikel können dann im erfindungsgemäßen Verfahren auch als "disposabel" bzw. "Single-Use"-Artikel bezeichnet werden ("SU Technologie"). Dadurch wird der keimreduzierte Zustand des erfindungsgemäßen Verfahrens und modularer Anlage noch weiter verbessert.

Der Begriff "Produktstrom" meint im Rahmen der Erfindung das zellfreie Fluid aus einer heterogenen Zellkultur-Fluid-Mischung, welche das Produkt enthält, sowie das Ergebnis jeder anderen Verfahrensschritte des erfindungsgemäßen Verfahrens, also den Produktstrom nach Filtration, nach Chromatographie, nach Virenabreicherung, nach Ultrafiltration, nach Diafiltration, oder nach weiteren Schritten des erfindungsgemäßen Verfahrens, wobei diese Produktströme dann unterschiedliche Konzentrationen und Reinheitsgrade aufweisen können.

Der Begriff "Virenabreicherung" meint im Rahmen der Erfindung eine Verringerung der Konzentration an aktiven Viren pro Volumeneinheit des zu behandelnden Fluids, bis hin zur vollständigen Inaktivierung und/oder Entfernung, der in dem zu behandelnden Fluid enthaltenen Viren.

Der Begriff "Bubble-Trap" meint im Rahmen der Erfindung eine Vorrichtung zum Auffangen von Gasblasen, wobei dabei das betreffende Fluid entgast wird.

Der Begriff "modular" meint im Rahmen der Erfindung, dass die einzelnen Schritte des erfindungsgemäße Verfahrens in separaten, miteinander verbundenen Modulen durchgeführt werden können, wobei die Module vorkonfiguriert und keimreduziert sind und geschlossen, in unterschiedlichen Kombinationen miteinander verschaltet werden können.

Der Begriff "modulare Anlage" meint im Rahmen der Erfindung eine Serie von miteinander verbundenen Modulen ("Units") zur Durchführung von mindestens zwei Downstream- und / oder Upstream-Schritten, in denen ein Fluid ("Produktstrom") befördert werden kann. Erfindungsgemäß sind die Units zur kontinuierlichen Durchführung eines Schrittes geeignet und können mit einem kontinuierlichen Fluidstrom ("Produktstrom") betrieben werden. Die einzelnen Module der "modularen Anlage" können dabei in jeder Kombination miteinander verschaltet werden.

Der Begriff "geschlossen" bedeutet im Rahmen der Erfindung die Fahrweise des erfindungsgemäßen Verfahrens und der erfindungsgemäßen modularen Anlage, welche so gefahren werden, dass das Produkt, welches mit diesem Verfahren und dieser modularen Anlage produziert und/oder aufbereitet wird, nicht an die Raumumgebung exponiert wird. In das erfindungsgemäße geschlossene Verfahren und die entsprechende erfindungsgemäße geschlossene modulare Anlage können von außen Materialien, Gegenstände, Puffer und dergleichen zugegeben werden, wobei aber diese Zugabe in einer solchen Weise erfolgt, dass eine Exposition des produzierten und/oder aufbereiteten Produktes an die Raumumgebung vermieden wird.

Die im Stand der Technik bekannten, üblichen Verfahren weisen eine Reihe von Nachteilen auf, welche im Folgenden behandelt werden.

Bekannte Verfahren zur Herstellung von biopharmazeutischen und biologischen Produkten umfassen üblicherweise folgende Produktionsschritte, die miteinander verschaltet werden:
1. Perfusionskultur
2. Zellrückhaltesystem,
   alternativ zu Schritt 1 und 2 kann auch eine Feedbatchkultur vorgesehen sein,
3. Zellabtrennung
4. Puffer- bzw. Medienaustausch bevorzugt mit Konzentrierung
5. BioBurden-Reduzierung bevorzugt durch Sterilfiltration
6. Capture Chromatographie

Üblicherweise werden weitere Schritte zur weiteren Reinigung des Produktstroms durchgeführt, insbesondere:
7. Virusinaktivierung
8. Neutralisation, und
9. Optional eine weitere Tiefenfiltration, BioBurden-Reduzierung (Sterilfiltration).

Angesichts der hohen Qualitätsstandards bei der Herstellung von Biopharmazeutika folgen üblicherweise darüber hinaus folgende Schritte:
10. Chromatographische Zwischen- und Feinreinigung
11. BioBurden Reduzierung z. B. Sterilfiltration
12. Virenfiltration
13. Pufferaustausch und bevorzugt Konzentrierung, und
14. Sterilfiltration.

Bei der oben beschriebenen Herstellung stellen Zellen in einem Fermenter mit Nährstofflösung ein biologisches Produkt her, etwa ein Protein, beispielsweise ein therapeutisches Protein. Die Nährstofflösung ist dabei auch ein ideales Wachstumsmedium für Mikroorganismen, wie Bakterien und Sporen, woraus sich ein Problem hinsichtlich des ungewünschten Wachstums solcher Mikroorganismen ergibt. Dieses ungewünschte Wachstum von Mikroorganismen wird besonders bei längeren Laufzeiten ein Problem, weil die Nährstofflösung mit ansteigender Laufzeit des Verfahrens mehr und mehr kontaminiert wird, bis hin zu einem exponentiellen Wachstum von Mikroorganismen und damit Totalverlust der betreffenden Charge an hergestelltem biologischen Produkt.

Um der Forderung an ein schnelles und flexibles Neubeschicken der Produktionsanlage unter Wahrung maximaler Sauberkeit und Keimfreiheit gerecht zu werden, erfreuen sich auf dem Markt Konzepte für eine kontinuierliche Produktion bevorzugt mit Einwegtechnologie eines ständig wachsenden Interesses.

Für längere Laufzeiten eines solchen Verfahrens von mehreren Stunden über Tage bis Wochen reichen aber gewöhnliche Maßnahmen zur Sanitisierung nicht, etwa die üblichen "Clean-in-Place" (CIP)-Maßnahmen, wie z.B. Sanitisierung durch 1M NaOH. Bei Laufzeiten ab mehreren Stunden haben solche gewöhnlichen Verfahren und Anlagen daher den Nachteil, dass sie hinsichtlich möglicher Kontamination und/oder möglichen Keimwachstums sehr anfällig sind.

In der Regel folgt z.B. in der Herstellung von monoklonalen Antikörpern dem ersten Chromatgraphieschritt mit einer Prot-A Säule eine Vireninaktivierung durch sauren pH <4 oder alkalischen pH >9. Dieser Schritt ist zeitkritisch, d.h. zur Vireninaktivierung darf eine gewisse Zeit, in der Regel <2h nicht unterschritten werden. Bei einer zu langen Verweilzeit, z.B. 4 Stunden, wird jedoch der Antikörper zerstört.

Es bestand daher ein Bedarf an einem Verfahren zur kontinuierlichen Reinigung eines Produktes aus einer heterogenen Zellkultur-Fluid-Mischung, welches durch seine weitgehende Keimarmut eine kontinuierliche Fahrweise von bis zu mehreren Wochen ermöglicht. Dabei stellt die kontinuierliche Vireninaktivierung in Kopplung mit einem vorgelagerten Chromatographieschritt eine besondere Herausforderung dar, da die Elution des Antikörpers immer absatzweise in diskreten Zeitschritten mit Flukuationen in Konzentration und pH Wert erfolgt.

Es war daher die Aufgabe der vorliegenden Erfindung, ein Verfahren und eine entsprechende Anlage zu entwickeln, mit dem ein Produkt, etwa ein Protein, kontinuierlich über einen Zeitraum von mehreren Stunden bis hin zu mehreren Wochen aus Produktstrom eluiert werden kann. Der kontinuierliche Elutionsstrom wird dann kontinuierlich von Viren abgereichert.

Die Erfindung löst diese Aufgabe durch Bereitstellen eines Verfahrens zur kontinuierlichen Elution eines biopharmazeutischen, biologischen, makromolekularen Produktes von mehr als einer Chromatographiesäule, umfassend die Schritte:
(a) Bereitstellen eines Produktstromes über einen Inlet,
(b) gleichzeitiges Beladen von n Front-Chromatographiesäulen mit dem Produktstrom in einer Beladungszone mit einer Beladungszeit t_{B}, wobei ein Ausgangsstrom der n Front-Chromatographiesäulen auf mindestens n-1 Recovery-Chromatographiesäulen in einer Recoveryzone gleichzeitig verteilt wird,
   wobei n zwischen 1 und 5 liegt, und
   wobei die n Front-Chromatographiesäulen zu einem gegebenen Zeitpunkt eine unterschiedlich lange Beladungszeit t_{B} zwischen 0 und L₁, zwischen L₁ und L₂, und bis zu zwischen Ln-1 und Ln aufweisen,
   wobei jede Front-Chromatographiesäule eine gesamte Beladungszeit von Lₙ aufweist,
   dadurch gekennzeichnet, dass periodisch nach einer konstanten Switch Zeit ts von Lₙ - Lₙ₋₁, die Front-Chromatographiesäulen n aus der Beladungszone heraustritt und eine Recovery-Chromatographiesäule aus der Recoveryzone in die Beladungszone eintritt,
(c) Waschen der mit Produkt beladenen Säule aus Schritt (b) mit mindestens einem Waschpuffer,
(d) Elution des Produktes von der gewaschenen Säule aus Schritt (c) mit einer Elutionszeit t_{E}, die ≥ 80% der Switch-Zeit ts ist, und
   dass sich über 80% der Gesamtlaufzeit t_{G} kontinuierlich mindestens eine Chromatographiesäule im Elutionsschritt (d) befindet.

Der technische Vorteil einer solchen kontinuierlichen Elution ist, dass in der Herstellung von sensitiven biopharmazeutischen, biologischen, makromolekularen Produkten, z.B. monoklonalen Antikörpern, die Verweilzeiten des Produktes in dem jeweiligen Verfahrensschritt minimiert werden. Denn üblicherweise folgt einem ersten Chromatgraphieschritt mit z.B. einer Protein-A Säule eine Vireninaktivierung durch sauren pH <4 oder alkalischen pH >9 folgt. Dieser Schritt ist zeitkritisch, d.h. zur Vireninaktivierung darf eine gewisse Zeit, in der Regel <2h nicht unterschritten werden. Bei einer zu langen Verweilzeit, z.B. 4 Stunden, wird jedoch der Antikörper zerstört.

Bevorzugt wird in dem erfindungsgemäßen Verfahrens nach dem Elutionsschritt (d) das eluierte Produkt durch einen Homogenisierungsschritt (e) vermischt, bevorzugt durch eine Rezyklierungsschleife mit einem Rezyklierungsbehälter, oder durch eine Rezyklierungsschleife ohne einen Rezyklierungsbehälter, oder durch einen single-use statischen Mischer.

In einer weiteren Ausführungsform des erfindungsgemäßen Verfahrens umfasst das Verfahren weiterhin den Schritt (f) Regeneration der eluierten Säule aus Schritt (d).

Die in dem erfindungsgemäßen Verfahren verwendeten Front-Chromatographiesäulen und Recovery-Chromatographiesäulen können jedes geeignete Bindungsprinzip aufweisen, etwa Affiniät des Produktes zu einem Liganden, ionische Wechselwirkungen, Metalchelat-Bindung, hydrophobe Interaktionen oder reine van-der-Waals-Kräfte.

Bevorzugt binden die Front-Chromatographiesäulen und die Recovery-Chromatographiesäulen Produkt nach dem Affinitätsprinzip, über ionische Wechselwirkungen, über Metalchelat-Bindung, über hydrophobe Interaktionen oder über van-der-Waals-Kräfte, wobei die Front-Chromatographiesäulen und die Recovery-Chromatographiesäulen bei Bindung nach dem Affinitätsprinzip einen Ligand umfassen, der bevorzugt ausgewählt ist aus der Gruppe bestehend aus Protein A, Protein G, Protein L, IgM, IgG und einem von Protein A, Protein G, Protein L, IgM und IgG unterschiedlichen rekombinanten Protein, welches eine Affinität für das Produkt hat.

Bevorzugt umfasst das biopharmazeutische, biologische makromolekulare Produkt ein Protein, Peptid oder eine DNA oder RNA, wobei das Protein oder Peptid ausgewählt ist aus der Gruppe bestehend aus monoklonalen Antikörpern, polyklonalen Antikörpern, rekombinanten Proteinen und Protein-Impfstoffen, und wobei die DNA oder RNA Teil eines DNA- und/oder RNA-Wirkstoffes oder -Impfstoffes ist.

In einer weiteren Ausführungsform des erfindungsgemäßen Verfahrens wird der pH Wert des eluierten Produktes aus Schritt (d) zusätzlich durch ein pH-Stellmittel eingestellt, bevorzugt vor dem Homogenisierungsschritt (e), oder während des Homogenisierungsschrittes (e).

Bevorzugt wird das homogenisierte Produkt aus Schritt (e) durch eine definierte Verweilzeitstrecke, bevorzugt einen Coiled-Flow Inverter (CFI), geleitet.

Bevorzugt beträgt die Gesamtlaufzeit t_{G} des Verfahrens mindestens 4 Stunden, bevorzugt mindestens 8 Stunden, bevorzugt mindestens 12 Stunden, bevorzugt mindestens 24 Stunden, weiterhin bevorzugt mindestens 48 Stunden, weiterhin bevorzugt mindestens 7 Tagen, weiterhin bevorzugt mindestens 4 Wochen, und besonders bevorzugt mindestens 8 Wochen auf. Eine derart lange Laufzeit von mehreren Wochen in bevorzugt kontinuierlicher Fahrweise kann insbesondere durch eine geschlossene, modulare und vor allem bevorzugt keimreduzierte Fahrweise des Verfahrens ermöglicht werden.

Bevorzugt werden die Schritte (a) bis (d), bevorzugt die Schritte (a) bis (f) keimreduziert durchgeführt, wobei bevorzugt alle eingesetzten, produkt-berührten Elemente, insbesondere die Front-Chromatographiesäulen und die Recovery-Chromatographiesäulen, durch eine geeignete Keimreduktionsmethode keimreduziert sind.

Derartige geeignete Keimreduktionsmethoden können ausgewählt sein aus der Gruppe bestehend aus Gamma-Bestrahlung, Beta-Bestrahlung, Autoklavieren, Ethylenoxid (ETO) Behandlung, Ozon-Behandlung (O₃), Wasserstoffperoxid-Behandlung (H₂O₂) und Steam-in-place (SIP) Behandlung.

Bevorzugt sind alle in dem Verfahren verwendeten Flüssigkeiten, Gase und Feststoffe keimreduziert, wobei die Keimreduktion bevorzugt durch eine Filtration durch einen Filter mit einer Porengröße von bevorzugt ≤ 0,45 µm erfolgt, und, bevorzugt während des Verfahrens keine Inprocess-Sterilisierung durchgeführt wird.

In einer weiteren Ausführungsform der Erfindung wird vor Schritt (b) ein Entgasen aller Fluide durchgeführt, welche auf die Chromatographiesäule kommen, wobei dass Entgasen vorzugsweise durch mindestens eine Bubble-Trap und/oder durch mindestens eine hydrophobe Mikrofiltrationsmembran über Vakuum und/oder durch Behandeln mit Ultraschall und/oder durch Durchperlen mit Helium erfolgt.

Die vorliegende Erfindung einschließlich bevorzugter Ausführungsformen wird in Verbindung mit den nachfolgenden Zeichnungen und Beispielen erläutert, ohne hierauf beschränkt zu sein. Die Ausführungsformen können beliebig miteinander kombiniert werden, sofern sich nicht eindeutig das Gegenteil aus dem Kontext ergibt.

Es zeigen:

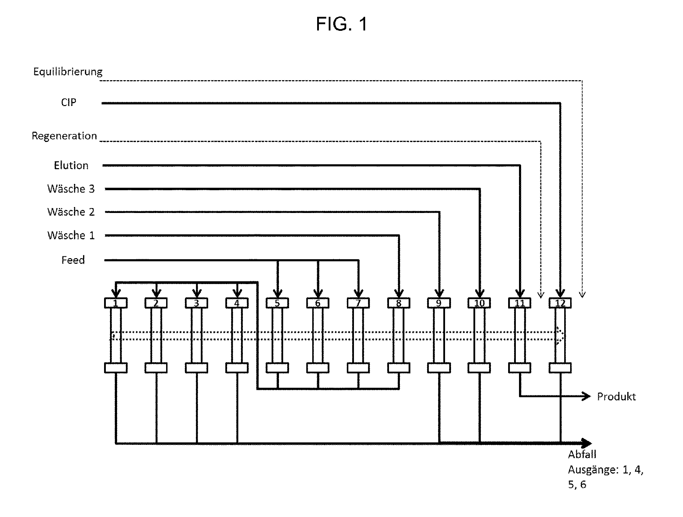

**Figur 1****:** Schematischer Aufbau der Eingangsströme und der Ausgangsströme in einer kontinuierlichen Chromatographieanlage mit 12 Säulen. Die Eingangsströme sind dabei wie in Beispiel 1 konfiguiert. Im Chromatographiezyklus bewegen sich die Säulen von links nach rechts, d.h. von der ersten Beladung in der Recoveryzone bis zum CIP und Equilibrieren. Zudem wird dargestellt, dass einzelne Prozessschritte wie z.B. Regeneration und Equilibrierung nicht kontinuierlich sein können.

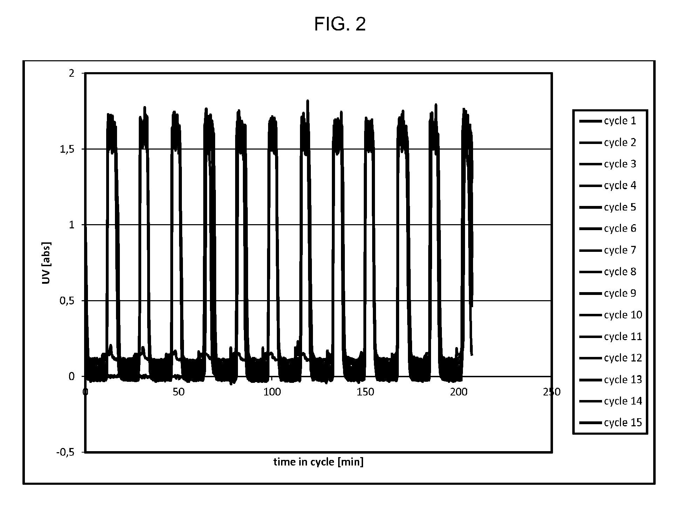

**Figur 2****:** UV-Absorption bei 280nm der Elutionspeaks während 15 Zyklen aus Beispiel 1. Das UV Signal ist dabei repräsentative für die Konzentration der Proteine in der Elution. Der Elutionsstrom ist konstant, die Proteinkonzentration ist jedoch periodisch.

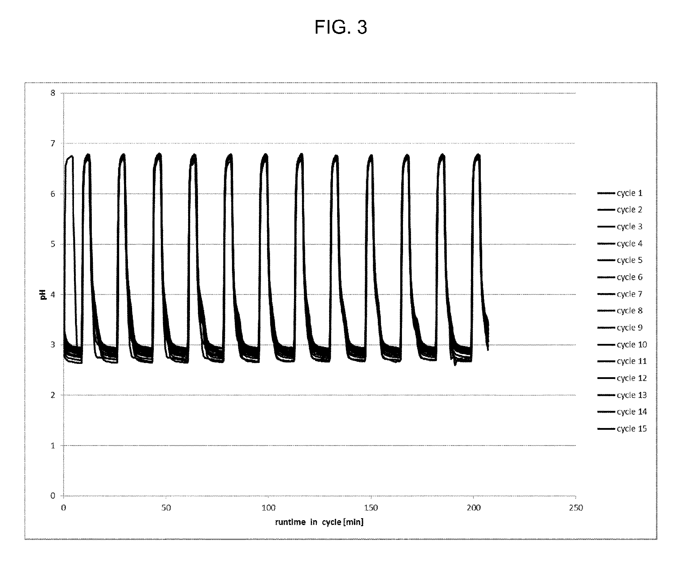

**Figur 3****:** pH Wert der Elutionspeaks während 15 Zyklen aus **Beispiel 1.** Der Elutionsstrom ist konstant, der pH Wert schwant jedoch periodisch, da der Puffer der Wäsche 3 zunächst verdrängt werden muss.
**Figur 4****:** Das Prinzip der kontinuierlichen Elution mit anschließender kontinuierlicher Virusinaktivierung. Dabei wird der fluktuieren kontinuierliche Elutionsstrom durch eine Homogenisierungsschleife vermischt und dann kontinuierlich der Verweilzeitstrecke zugeführt.
   1 Kontinuierlicher Elutionsstrom mit pH Fluktuation
   2 Homogenisierungsschleife
   3 CFI (Cold-Flow-Inverter) Verweilzeitstrecke

### Beispiel 1

Im Beispiel 1 wurde ein monoklonaler Antikörper IgG1 verwendet. Die Fermenterbrühe wurde über ein Fed-batch Verfahren hergestellt. Die Feedkonzentration entsprach 0,8-1,5 g/L. Als Chromatographie System wurde ein Tarpon System verwendet. Dabei wurden 12 Säulen mit einem Innendurchmesser von 16mm eingesetzt. Die Säulen wurden mit dem Protein A Harz MabselectSure der Firma GE gepackt. Dabei wurden die Säulen gemäß Herstellerangaben zu einer Länge von 8 cm mit einem Volumen von 16.1 mL gepackt.
Das Tarpon System hatte 8 Eingänge. Die Belegung der Eingänge war wie folgt:
Eingang 1 : Verbindung des Ausgangs der Frontsäulen
Eingang 2: Feed Fermenterbrühe
Eingang3: Puffer Wäsche 1
Eingang 4: Puffer Wäsche 2
Eingang 5: Elutionspuffer
Eingang 6: Regenerationspuffer
Eingang 7: Cleaning in Place (CIP)
Eingang 8: Equilibrierungspuffer bzw. Wäsche 3 Puffer

Die Ausgänge des Chromatographiesystems waren wie folgt.
Ausgang 1: Abfallstrom
Ausgang 2: geschlossen
Ausgang 3: Elution
Ausgang 4: Abfall der Wäsche
Ausgang 5: Abfall des Flowthroughs aus der Recovery Zone
Ausgang 6: Ausgang der Frontsäulen verbunden mit Eingang 1

Die Eingänge 2-6 wurden jeweils über separate Kolbenpumpen mit den jeweiligen Lösungen / Puffern versorgt.
Die Puffersysteme waren wie folgt zusammengesetzt:
Wäsche 1: 50mM Tris, 2.5M NaCl pH7
Wäsche 2: 50mM NaAc, 1M NaCl, pH5
Elutionspuffer: 100mM NaAcetat, 50 mM NaCl, pH 5
Regenerationspuffer: 50mM Na Actat, 500 mM NaCl, pH 2.7
Wäsche 3 /Equibrierungspuffer: 50mM Tris / 50 mM NaCl pH 7
CIP: 0.5M NaOH

Jede Frontsäule wurde in der Beladungszone mit 32.25 Säulenvolumina (SV) mit einer Feedrate von 30 mL / min beladen. Dabei waren immer drei Säulen gleichzeitig in der Beladungszone während sich jeweils 4 Recovery Säulen in der Recoveryzone befanden, die ungebundenen IgG aus der Beladungszone auffangen konnten. Die Switch Zeit betrug 17,29 min.

Nachdem eine Frontsäule für 3 Switchzeiten beladen wurde, wurde diese Säule innerhalb von einer Switch Zeit mit 10 SV in der Wäsche 1 gewaschen. Danach wurde die Säule ebenfalls mit 10 SV in der Wäsche 2 innerhalb einer Switch Zeit gewaschen. Die Wäsche 3 reduzierte danach innerhalb einer halben Switch Zeit mit 5 SV die Salzfracht auf der gewaschenen Säule.

Die gewaschene Säule wurde daraufhin in genau einer Switch Zeit mit 4.5 SV eluiert, wodurch während des gesamten Prozesses ein kontinuierlicher Elutionsstrom mit ∼4.2 mL/min realisiert wurde. Die Regeneration, CIP und Equilibrierung der Säule erfolgte dann jeweils innerhalb einer halben Switch Zeit mit einem Aufgabevolumen von 5, 3 und 5 SV.

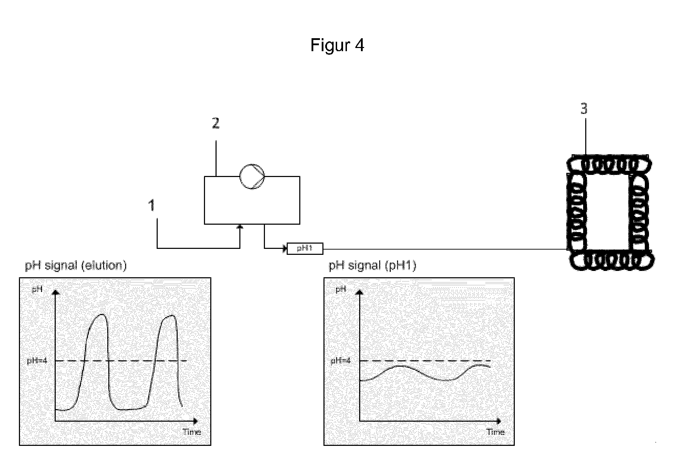

Der kontinuierliche Elutionsstrom wurde mit einer Flussrate von 4,2 mL/min einer Homogenisierungsschleife zugeführt. Der pH Wert in der Elution schwankte dabei von 3.1 bis 6,6 wie in Figur 4 schematisiert. Für die Vireninaktivierung war ein pH Wert < 4 erforderlich um eine effektive Vireninaktivierung zu erreichen. Die starken pH Schwankungen wurden durch die Homogenisierung auf einen maximalen pH Wert von 3.9 gedämpft wie in Figur 4 dargestellt. Die Homogenisierungsschleife bestand aus einem Schlauch mit einem Innendurchmesser von 6.4mm und einem Volumen von 30ml. Dabei pumpte eine Schlauchpumpe den Inhalt mit einer Flussrate von 380 mL/min um. Das Risiko einer Kurzschlussströmung wurde dadurch minimiert, dass die Strömungsrichtung entgegen der Richtung von Eingang nach Ausgang verlief. Das homogenisierte Produkte strömte mit der gleichen Flussrate wie die Elution aus der Homogenisierungsschleife. Dieses wurde nun in die Verweilzeitschleife geleitet. Die Verweilzeitschleife war ein Coiled-Flow Inverter (CFI) mit einer engen Verweilzeitverteilung, die vergleichbar mit der einer idealen Pfropfenströmung ist. Die minimale Verweilzeit im CFI betrug 60 min. Der Aufbau des CFI ist in Klutz et al. (Klutz, S., Kurt, S.K., Lobedann, M., Kockmann, N., 2015) und in "Narrow residence time distribution in tubular reactor concept for Reynolds number range of 10-100, Chem. Eng. Res. Des. 95, 22-33" beschrieben. Die technischen Daten sind in Tabelle 1 aufgeführt.

**Tabelle 1: Design Parameter des CFI Reaktors für kontinuierlich gefahrene Vireninaktivierung**

| **Design-Variable** | **Wert** | **Einheit** |
|---|---|---|
| Schlauchinnendurchmesser | 3,2 | mm |
| Schlauchwandstärke | 1,6 | mm |
| Durchmesser Rahmen (Coil) | 63 | mm |
| Schlauchabstand in Windung (minimum) | 6,4 | mm |
| Anzahl der Windung pro gerader Rahmenseite | 9 | - |
| Anzahl der geraden Rahmenseiten | 20 | - |
| Zahl der 90°-Knicke | 19 | - |
| Volumetrische Flussrate | 4.2 | mL min⁻¹ |
| Schlauchlänge (Sanipure^{®} tube) | 40 | m |
| Schlauch Holdup volumen | 322 | mL |

Die Arbeiten, die zu dieser Anmeldung geführt haben, wurden gemäß der Finanzhilfevereinbarung "Bio.NRW: MoBiDiK - Modulare Bioproduktion - Disposable und Kontinuierlich" im Rahmen des Europäischen Fonds für regionale Entwicklung (EFRE) gefördert.

## Patentansprüche

1. Verfahren zur kontinuierlichen Elution eines biopharmazeutischen, biologischen, makromolekularen Produktes von mehr als einer Chromatographiesäule, umfassend die Schritte:
(a) Bereitstellen eines Produktstromes über einen Inlet,
(b) gleichzeitiges Beladen von n Front-Chromatographiesäulen mit dem Produktstrom in einer Beladungszone mit einer Beladungszeit t_{B}, wobei ein Ausgangsstrom der n Front-Chromatographiesäulen auf mindestens n-1 Recovery-Chromatographiesäulen in einer Recoveryzone gleichzeitig verteilt wird,
wobei n zwischen 1 und 5 liegt, und
wobei die n Front-Chromatographiesäulen zu einem gegebenen Zeitpunkt eine unterschiedlich lange Beladungszeit t_{B} zwischen 0 und L₁, zwischen L₁ und L₂, und bis zu zwischen Ln-1 und Ln aufweisen,
wobei jede Front-Chromatographiesäule eine gesamte Beladungszeit von Lₙ aufweist, **dadurch gekennzeichnet, dass** periodisch nach einer konstanten Switch Zeit ts von Lₙ - Lₙ₋₁, die Front-Chromatographiesäulen n aus der Beladungszone heraustritt und eine Recovery-Chromatographiesäule aus der Recoveryzone in die Beladungszone eintritt,
(c) Waschen der mit Produkt beladenen Säule aus Schritt (b) mit mindestens einem Waschpuffer,
(d) Elution des Produktes von der gewaschenen Säule aus Schritt (c) mit einer Elutionszeit t_{E}, die ≥ 80% der Switch-Zeit ts ist, und
dass sich über 80% der Gesamtlaufzeit t_{G} kontinuierlich mindestens eine Chromatographiesäule im Elutionsschritt (d) befindet.

2. Verfahren nach Anspruch 1, **dadurch gekennzeichnet, dass** nach dem Elutionsschritt (d) das eluierte Produkt durch einen Homogenisierungsschritt (e) vermischt wird, bevorzugt durch eine Rezyklierungsschleife mit einem Rezyklierungsbehälter, oder durch eine Rezyklierungsschleife ohne einen Rezyklierungsbehälter, oder durch einen single-use statischen Mischer.

3. Verfahren nach Anspruch 1 oder Anspruch 2, **dadurch gekennzeichnet, dass** das Verfahren weiterhin den Schritt
(f) Regeneration der eluierten Säule aus Schritt (d)
umfasst.

4. Verfahren nach einem der Ansprüche 1 bis 3, **dadurch gekennzeichnet, dass** die Front-Chromatographiesäulen und die Recovery-Chromatographiesäulen Produkt nach dem Affinitätsprinzip, über ionische Wechselwirkungen, über Metalchelat-Bindung, über hydrophobe Interaktionen oder über van-der-Waals-Kräfte binden, wobei die Front-Chromatographiesäulen und die Recovery-Chromatographiesäulen bei Bindung nach dem Affinitätsprinzip einen Ligand umfassen, der bevorzugt ausgewählt ist aus der Gruppe bestehend aus Protein A, Protein G, Protein L, IgM, IgG und einem von Protein A, Protein G, Protein L, IgM und IgG unterschiedlichen rekombinanten Protein, welches eine Affinität für das Produkt hat.

5. Verfahren nach einem der Ansprüche 1 bis 4, **dadurch gekennzeichnet, dass** das biopharmazeutische, biologische makromolekulare Produkt ein Protein, Peptid ist oder eine DNA oder RNA umfasst, wobei das Protein oder Peptid ausgewählt ist aus der Gruppe bestehend aus monoklonalen Antikörpern, polyklonalen Antikörpern, rekombinanten Proteinen und Protein-Impfstoffen, und wobei die DNA oder RNA Teil eines DNA- und/oder RNA-Wirkstoffes oder -Impfstoffes ist.

6. Verfahren nach einem der Ansprüche 1 bis 5, **dadurch gekennzeichnet, dass** der pH Wert des eluierten Produktes aus Schritt (d) zusätzlich durch ein pH-Stellmittel eingestellt wird, bevorzugt vor dem Homogenisierungsschritt (e), oder während des Homogenisierungsschrittes (e).

7. Verfahren nach 1 bis 6 **dadurch gekennzeichnet, dass** das homogenisierte Produkt aus Schritt (e) durch eine definierte Verweilzeitstrecke, bevorzugt einen Coiled-Flow Inverter (CFI), geleitet wird.

8. Verfahren nach einem der Ansprüche 1 bis 7, **dadurch gekennzeichnet, dass** die Gesamtlaufzeit t_{G} des Verfahrens mindestens 4 Stunden, bevorzugt mindestens 8 Stunden, bevorzugt mindestens 12 Stunden, bevorzugt mindestens 24 Stunden, weiterhin bevorzugt mindestens 48 Stunden, weiterhin bevorzugt mindestens 7 Tage, weiterhin bevorzugt mindestens 4 Wochen, und besonders bevorzugt mindestens 8 Wochen beträgt.

9. Verfahren nach einem der Ansprüche 1 bis 8, **dadurch gekennzeichnet, dass** die Schritte (a) bis (d), bevorzugt die Schritte (a) bis (f) keimreduziert durchgeführt werden, wobei bevorzugt alle eingesetzten, produkt-berührten Elemente, insbesondere die Front-Chromatographiesäulen und die Recovery-Chromatographiesäulen, durch eine geeignete Keimreduktionsmethode keimreduziert sind.

10. Verfahren nach Anspruch 9, **dadurch gekennzeichnet, dass** die geeignete Keimreduktionsmethode ausgewählt ist aus der Gruppe bestehend aus Gamma-Bestrahlung, Beta-Bestrahlung, Autoklavieren, Ethylenoxid (ETO) Behandlung, Ozon-Behandlung (O₃), Wasserstoffperoxid-Behandlung (H₂O₂) und Steam-in-place (SIP) Behandlung.

11. Verfahren nach einem der Ansprüche 1 bis 10, **dadurch gekennzeichnet, dass** alle in dem Verfahren verwendeten Flüssigkeiten, Gase und Feststoffe keimreduziert sind, wobei die Keimreduktion bevorzugt durch eine Filtration durch einen Filter mit einer Porengröße von bevorzugt ≤ 0,45 µm erfolgt, und, dass bevorzugt während des Verfahrens keine Inprocess-Sterilisierung durchgeführt wird.

12. Verfahren nach Anspruch 1 bis 11, **dadurch gekennzeichnet, dass** vor Schritt (b) ein Entgasen aller Fluide durchgeführt wird, welche auf die Chromatographiesäule kommen, wobei dass Entgasen vorzugsweise durch mindestens eine Bubble-Trap und/oder durch mindestens eine hydrophobe Mikrofiltrationsmembran über Vakuum und/oder durch Behandeln mit Ultraschall und/oder durch Durchperlen mit Helium erfolgt.
